# EUROPEAN PATENT APPLICATION

(11) **EP 2 992 898 A1**
(43) Date of publication of application: **09.03.2016**
(21) Application number: 14183505.8
(22) Date of filing: 04.09.2014
(51) Int. Cl.: A61K 39/39, C07K 14/54, C07K 14/715

(54) **T-cell adjuvant and its use for therapeutic or prophylactic vaccination**

(71) Applicant: Klinikum rechts der Isar der Technischen Universität München, 81675 München (DE)
(72) Inventor: Knolle, Percy, 81735 München (DE)
(74) Representative: Aisch, Sebastian

(57) **Abstract**

The present invention relates in a first aspect to a polypeptide complex comprising IL-6 and a soluble IL-6 receptor or a nucleic acid sequence encoding the same for use as an adjuvant. It has been recognized that the polypeptide complex comprising IL-6 and a soluble IL-6 receptor, in particular, Hyper-IL-6, represents an adjuvant directly acting on T-cells. It is submitted that the polypeptide or a nucleic acid sequence encoding the same according to the present invention represents the first direct acting T-cell adjuvant described in the art. In another aspect, the present invention relates to a pharmaceutical composition comprising the polypeptide complex or a nucleic acid sequence encoding the same comprising IL-6 and a soluble IL-6 receptor according to the present invention as an adjuvant, a pharmaceutical active ingredient and, optionally, additional components whereby the pharmaceutical composition is a therapeutic or prophylactic vaccine. Finally, the present invention relates to the use of the polypeptide or a nucleic acid sequence encoding the same according to the present invention comprising IL-6 and a soluble IL-6 receptor for activation of effector and memory T-cells in a subject, in particular, in a human. Particularly, the T-cells are cytotoxic T-cells, CD8⁺ T-cells.

SEQUENCE LISTING

<110>  Klinikum rechts der Isar der Technischen Universitaet
       Muenchen

<120>  T-cell Adjuvant and its use for therapeutic or prophylactic
       vaccination

<130>  5632-001 EP-1

<160>  5

<170>  PatentIn version 3.5

<210>  1
<211>  1612
<212>  DNA
<213>  artificial

<220>
<223>  Synthetic polypeptide

<220>
<221>  CDS
<222>  (1)..(1593)

<400>  1
gtc gac gca tgg agt ggt agc cga gga gga agc atg ctg gcc gtc ggc      48
Val Asp Ala Trp Ser Gly Ser Arg Gly Gly Ser Met Leu Ala Val Gly
1               5                   10                  15

tgc gcg ctg ctg gct gcc ctg ctg gcc gcg ccg gga gcg gcg ctg gcc      96
Cys Ala Leu Leu Ala Ala Leu Leu Ala Ala Pro Gly Ala Ala Leu Ala
            20                  25                  30

cca agg cgc tgc cct gcg cag gag gtg gca aga ggc gtg ctg acc agt      144
Pro Arg Arg Cys Pro Ala Gln Glu Val Ala Arg Gly Val Leu Thr Ser
        35                  40                  45

ctg cca gga gac agc gtg act ctg acc tgc ccg ggg gta gag ccg gaa      192
Leu Pro Gly Asp Ser Val Thr Leu Thr Cys Pro Gly Val Glu Pro Glu
    50                  55                  60

gac aat gcc act gtt cac tgg gtg ctc agg aag ccg gct gca ggc tcc      240
Asp Asn Ala Thr Val His Trp Val Leu Arg Lys Pro Ala Ala Gly Ser
65                  70                  75                  80

cac ccc agc aga tgg gct ggc atg gga agg agg ctg ctg ctg agg tcg      288
His Pro Ser Arg Trp Ala Gly Met Gly Arg Arg Leu Leu Leu Arg Ser
                85                  90                  95

gtg cag ctc cac gac tct gga aac tat tca tgc tac cgg gcc ggc cgc      336
Val Gln Leu His Asp Ser Gly Asn Tyr Ser Cys Tyr Arg Ala Gly Arg
            100                 105                 110

cca gct ggg act gtg cac ttg ctg gtg gat gtt ccc ccc gag gag ccc      384
Pro Ala Gly Thr Val His Leu Leu Val Asp Val Pro Pro Glu Glu Pro
        115                 120                 125

cag ctc tcc tgc ttc cgg aag agc ccc ctc agc aat gtt gtt tgt gag      432
Gln Leu Ser Cys Phe Arg Lys Ser Pro Leu Ser Asn Val Val Cys Glu
    130                 135                 140

tgg ggt cct cgg agc acc cca tcc ctg acg aca aag gct gtg ctc ttg     480
Trp Gly Pro Arg Ser Thr Pro Ser Leu Thr Thr Lys Ala Val Leu Leu
145                 150                 155                 160

gtg agg aag ttt cag aac agt ccg gcc gaa gac ttc cag gag ccg tgc     528
Val Arg Lys Phe Gln Asn Ser Pro Ala Glu Asp Phe Gln Glu Pro Cys
                165                 170                 175

cag tat tcc cag gag tcc cag aag ttc tcc tgc cag tta gca gtc ccg     576
Gln Tyr Ser Gln Glu Ser Gln Lys Phe Ser Cys Gln Leu Ala Val Pro
            180                 185                 190

gag gga gac agc tct ttc tac ata gtg tcc atg tgc gtc gcc agt agt     624
Glu Gly Asp Ser Ser Phe Tyr Ile Val Ser Met Cys Val Ala Ser Ser
        195                 200                 205

gtc ggg agc aag ttc agc aaa act caa acc ttt cag ggt tgt gga atc     672
Val Gly Ser Lys Phe Ser Lys Thr Gln Thr Phe Gln Gly Cys Gly Ile
    210                 215                 220

ttg cag cct gat ccg cct gcc aac atc aca gtc act gcc gtg gcc aga     720
Leu Gln Pro Asp Pro Pro Ala Asn Ile Thr Val Thr Ala Val Ala Arg
225                 230                 235                 240

aac ccc cgc tgg ctc agt gtc acc tgg caa gac ccc cac tcc tgg aac     768
Asn Pro Arg Trp Leu Ser Val Thr Trp Gln Asp Pro His Ser Trp Asn
                245                 250                 255

tca tct ttc tac aga cta cgg ttt gag ctc aga tat cgg gct gaa cgg     816
Ser Ser Phe Tyr Arg Leu Arg Phe Glu Leu Arg Tyr Arg Ala Glu Arg
            260                 265                 270

tca aag aca ttc aca aca tgg atg gtc aag gac ctc cag cat cac tgt     864
Ser Lys Thr Phe Thr Thr Trp Met Val Lys Asp Leu Gln His His Cys
        275                 280                 285

gtc atc cac gac gcc tgg agc ggc ctg agg cac gtg gtg cag ctt cgt     912
Val Ile His Asp Ala Trp Ser Gly Leu Arg His Val Val Gln Leu Arg
    290                 295                 300

gcc cag gag gag ttc ggg caa ggc gag tgg agc gag tgg agc ccg gag     960
Ala Gln Glu Glu Phe Gly Gln Gly Glu Trp Ser Glu Trp Ser Pro Glu
305                 310                 315                 320

gcc atg ggc acg cct tgg aca gaa tcc agg agt cct cca gct cga gga     1008
Ala Met Gly Thr Pro Trp Thr Glu Ser Arg Ser Pro Pro Ala Arg Gly
                325                 330                 335

ggt gga ggt tct gga ggt gga ggt tct gtc gag cca gta ccc cca gga     1056
Gly Gly Gly Ser Gly Gly Gly Gly Ser Val Glu Pro Val Pro Pro Gly
            340                 345                 350

gaa gat tcc aaa gat gta gcc gcc cca cac aga cag cca ctc acc tct     1104
Glu Asp Ser Lys Asp Val Ala Ala Pro His Arg Gln Pro Leu Thr Ser
        355                 360                 365

tca gaa cga att gac aaa caa att cgg tac atc ctc gac ggc atc tca     1152
Ser Glu Arg Ile Asp Lys Gln Ile Arg Tyr Ile Leu Asp Gly Ile Ser
    370                 375                 380

gcc ctg aga aag gag aca tgt aac aag agt aac atg tgt gaa agc agc     1200
Ala Leu Arg Lys Glu Thr Cys Asn Lys Ser Asn Met Cys Glu Ser Ser

385                 390                 395                 400

aaa gag gca ctg gca gaa aac aac ctg aac ctt cca aag atg gct gaa      1248
Lys Glu Ala Leu Ala Glu Asn Asn Leu Asn Leu Pro Lys Met Ala Glu
                405                 410                 415

aaa gat gga tgc ttc caa tct gga ttc aat gag gag act tgc ctg gtg      1296
Lys Asp Gly Cys Phe Gln Ser Gly Phe Asn Glu Glu Thr Cys Leu Val
            420                 425                 430

aaa atc atc act ggt ctt ttg gag ttt gag gta tac cta gag tac ctc      1344
Lys Ile Ile Thr Gly Leu Leu Glu Phe Glu Val Tyr Leu Glu Tyr Leu
        435                 440                 445

cag aac aga ttt gag agt agt gag gaa caa gcc aga gct gtg cag atg      1392
Gln Asn Arg Phe Glu Ser Ser Glu Glu Gln Ala Arg Ala Val Gln Met
    450                 455                 460

agt aca aaa gtc ctg atc cag ttc ctg cag aaa aag gca aag aat cta      1440
Ser Thr Lys Val Leu Ile Gln Phe Leu Gln Lys Lys Ala Lys Asn Leu
465                 470                 475                 480

gat gca ata acc acc cct gac cca acc aca aat gcc agc ctg ctg acg      1488
Asp Ala Ile Thr Thr Pro Asp Pro Thr Thr Asn Ala Ser Leu Leu Thr
                485                 490                 495

aag ctg cag gca cag aac cag tgg ctg cag gac atg aca act cat ctc      1536
Lys Leu Gln Ala Gln Asn Gln Trp Leu Gln Asp Met Thr Thr His Leu
            500                 505                 510

att ctg cgc agc ttt aag gag ttc ctg cag tcc agc ctg agg gct ctt      1584
Ile Leu Arg Ser Phe Lys Glu Phe Leu Gln Ser Ser Leu Arg Ala Leu
        515                 520                 525

cgg caa atg tagcatgggc accgtcgac                                     1612
Arg Gln Met
    530

<210>  2
<211>  531
<212>  PRT
<213>  artificial

<220>
<223>  Synthetic Construct

<400>  2

Val Asp Ala Trp Ser Gly Ser Arg Gly Gly Ser Met Leu Ala Val Gly
1               5                   10                  15

Cys Ala Leu Leu Ala Ala Leu Leu Ala Ala Pro Gly Ala Ala Leu Ala
            20                  25                  30

Pro Arg Arg Cys Pro Ala Gln Glu Val Ala Arg Gly Val Leu Thr Ser
        35                  40                  45

Leu Pro Gly Asp Ser Val Thr Leu Thr Cys Pro Gly Val Glu Pro Glu
    50                  55                  60

Asp Asn Ala Thr Val His Trp Val Leu Arg Lys Pro Ala Ala Gly Ser
65                  70                  75                  80

His Pro Ser Arg Trp Ala Gly Met Gly Arg Arg Leu Leu Leu Arg Ser
                85                  90                  95

Val Gln Leu His Asp Ser Gly Asn Tyr Ser Cys Tyr Arg Ala Gly Arg
            100                 105                 110

Pro Ala Gly Thr Val His Leu Leu Val Asp Val Pro Pro Glu Glu Pro
        115                 120                 125

Gln Leu Ser Cys Phe Arg Lys Ser Pro Leu Ser Asn Val Val Cys Glu
    130                 135                 140

Trp Gly Pro Arg Ser Thr Pro Ser Leu Thr Thr Lys Ala Val Leu Leu
145                 150                 155                 160

Val Arg Lys Phe Gln Asn Ser Pro Ala Glu Asp Phe Gln Glu Pro Cys
                165                 170                 175

Gln Tyr Ser Gln Glu Ser Gln Lys Phe Ser Cys Gln Leu Ala Val Pro
            180                 185                 190

Glu Gly Asp Ser Ser Phe Tyr Ile Val Ser Met Cys Val Ala Ser Ser
        195                 200                 205

Val Gly Ser Lys Phe Ser Lys Thr Gln Thr Phe Gln Gly Cys Gly Ile
    210                 215                 220

Leu Gln Pro Asp Pro Pro Ala Asn Ile Thr Val Thr Ala Val Ala Arg
225                 230                 235                 240

Asn Pro Arg Trp Leu Ser Val Thr Trp Gln Asp Pro His Ser Trp Asn
                245                 250                 255

Ser Ser Phe Tyr Arg Leu Arg Phe Glu Leu Arg Tyr Arg Ala Glu Arg
            260                 265                 270

Ser Lys Thr Phe Thr Thr Trp Met Val Lys Asp Leu Gln His His Cys
        275                 280                 285

Val Ile His Asp Ala Trp Ser Gly Leu Arg His Val Val Gln Leu Arg
    290                 295                 300

Ala Gln Glu Glu Phe Gly Gln Gly Glu Trp Ser Glu Trp Ser Pro Glu

305                 310                 315                 320

Ala Met Gly Thr Pro Trp Thr Glu Ser Arg Ser Pro Pro Ala Arg Gly
                325                 330                 335

Gly Gly Gly Ser Gly Gly Gly Gly Ser Val Glu Pro Val Pro Pro Gly
            340                 345                 350

Glu Asp Ser Lys Asp Val Ala Ala Pro His Arg Gln Pro Leu Thr Ser
        355                 360                 365

Ser Glu Arg Ile Asp Lys Gln Ile Arg Tyr Ile Leu Asp Gly Ile Ser
    370                 375                 380

Ala Leu Arg Lys Glu Thr Cys Asn Lys Ser Asn Met Cys Glu Ser Ser
385                 390                 395                 400

Lys Glu Ala Leu Ala Glu Asn Asn Leu Asn Leu Pro Lys Met Ala Glu
                405                 410                 415

Lys Asp Gly Cys Phe Gln Ser Gly Phe Asn Glu Glu Thr Cys Leu Val
            420                 425                 430

Lys Ile Ile Thr Gly Leu Leu Glu Phe Glu Val Tyr Leu Glu Tyr Leu
        435                 440                 445

Gln Asn Arg Phe Glu Ser Ser Glu Glu Gln Ala Arg Ala Val Gln Met
    450                 455                 460

Ser Thr Lys Val Leu Ile Gln Phe Leu Gln Lys Lys Ala Lys Asn Leu
465                 470                 475                 480

Asp Ala Ile Thr Thr Pro Asp Pro Thr Thr Asn Ala Ser Leu Leu Thr
                485                 490                 495

Lys Leu Gln Ala Gln Asn Gln Trp Leu Gln Asp Met Thr Thr His Leu
            500                 505                 510

Ile Leu Arg Ser Phe Lys Glu Phe Leu Gln Ser Ser Leu Arg Ala Leu
        515                 520                 525

Arg Gln Met
    530

<210>  3
<211>  184
<212>  PRT
<213>  homo sapiens

<400>  3

Pro Val Pro Pro Gly Glu Asp Ser Lys Asp Val Ala Ala Pro His Arg
1               5                   10                  15

Gln Pro Leu Thr Ser Ser Glu Arg Ile Asp Lys Gln Ile Arg Tyr Ile
            20                  25                  30

Leu Asp Gly Ile Ser Ala Leu Arg Lys Glu Thr Cys Asn Lys Ser Asn
        35                  40                  45

Met Cys Glu Ser Ser Lys Glu Ala Leu Ala Glu Asn Asn Leu Asn Leu
    50                  55                  60

Pro Lys Met Ala Glu Lys Asp Gly Cys Phe Gln Ser Gly Phe Asn Glu
65                  70                  75                  80

Glu Thr Cys Leu Val Lys Ile Ile Thr Gly Leu Leu Glu Phe Glu Val
                85                  90                  95

Tyr Leu Glu Tyr Leu Gln Asn Arg Phe Glu Ser Ser Glu Glu Gln Ala
            100                 105                 110

Arg Ala Val Gln Met Ser Thr Lys Val Leu Ile Gln Phe Leu Gln Lys
        115                 120                 125

Lys Ala Lys Asn Leu Asp Ala Ile Thr Thr Pro Asp Pro Thr Thr Asn
    130                 135                 140

Ala Ser Leu Leu Thr Lys Leu Gln Ala Gln Asn Gln Trp Leu Gln Asp
145                 150                 155                 160

Met Thr Thr His Leu Ile Leu Arg Ser Phe Lys Glu Phe Leu Gln Ser
                165                 170                 175

Ser Leu Arg Ala Leu Arg Gln Met
            180

<210>  4
<211>  323
<212>  PRT
<213>  homo sapiens

<400>  4

Met Leu Ala Val Gly Cys Ala Leu Leu Ala Ala Leu Leu Ala Ala Pro
1               5                   10                  15

Gly Ala Ala Leu Ala Pro Arg Arg Cys Pro Ala Gln Glu Val Ala Arg

20                  25                  30

Gly Val Leu Thr Ser Leu Pro Gly Asp Ser Val Thr Leu Thr Cys Pro
        35                  40                  45

Gly Val Glu Pro Glu Asp Asn Ala Thr Val His Trp Val Leu Arg Lys
    50                  55                  60

Pro Ala Ala Gly Ser His Pro Ser Arg Trp Ala Gly Met Gly Arg Arg
65                  70                  75                  80

Leu Leu Leu Arg Ser Val Gln Leu His Asp Ser Gly Asn Tyr Ser Cys
                85                  90                  95

Tyr Arg Ala Gly Arg Pro Ala Gly Thr Val His Leu Leu Val Asp Val
            100                 105                 110

Pro Pro Glu Glu Pro Gln Leu Ser Cys Phe Arg Lys Ser Pro Leu Ser
        115                 120                 125

Asn Val Val Cys Glu Trp Gly Pro Arg Ser Thr Pro Ser Leu Thr Thr
    130                 135                 140

Lys Ala Val Leu Leu Val Arg Lys Phe Gln Asn Ser Pro Ala Glu Asp
145                 150                 155                 160

Phe Gln Glu Pro Cys Gln Tyr Ser Gln Glu Ser Gln Lys Phe Ser Cys
                165                 170                 175

Gln Leu Ala Val Pro Glu Gly Asp Ser Ser Phe Tyr Ile Val Ser Met
            180                 185                 190

Cys Val Ala Ser Ser Val Gly Ser Lys Phe Ser Lys Thr Gln Thr Phe
        195                 200                 205

Gln Gly Cys Gly Ile Leu Gln Pro Asp Pro Pro Ala Asn Ile Thr Val
    210                 215                 220

Thr Ala Val Ala Arg Asn Pro Arg Trp Leu Ser Val Thr Trp Gln Asp
225                 230                 235                 240

Pro His Ser Trp Asn Ser Ser Phe Tyr Arg Leu Arg Phe Glu Leu Arg
                245                 250                 255

Tyr Arg Ala Glu Arg Ser Lys Thr Phe Thr Thr Trp Met Val Lys Asp
            260                 265                 270

Leu Gln His His Cys Val Ile His Asp Ala Trp Ser Gly Leu Arg His
        275                 280                 285

Val Val Gln Leu Arg Ala Gln Glu Glu Phe Gly Gln Gly Glu Trp Ser
    290                 295                 300

Glu Trp Ser Pro Glu Ala Met Gly Thr Pro Trp Thr Glu Ser Arg Ser
305                 310                 315                 320

Pro Pro Ala

<210>  5
<211>  13
<212>  PRT
<213>  artificial

<220>
<223>  synthetic polypeptide

<400>  5

Arg Gly Gly Gly Gly Ser Gly Gly Gly Gly Ser Val Glu
1               5                   10

## Description

The present invention relates in a first aspect to a polypeptide complex comprising IL-6 and a soluble IL-6 receptor or a nucleic acid sequence encoding the same for use as an adjuvant. It has been recognized that the polypeptide complex comprising IL-6 and a soluble IL-6 receptor or a nucleic acid sequence encoding the same, in particular, Hyper-IL-6, represents an adjuvant directly acting on T-cells. It is submitted that the polypeptide or a nucleic acid sequence encoding the same according to the present invention represents the first direct acting T-cell adjuvant described in the art. In another aspect, the present invention relates to a pharmaceutical composition comprising the polypeptide complex comprising IL-6 and a soluble IL-6 receptor or a nucleic acid sequence encoding the same according to the present invention as an adjuvant, a pharmaceutical active ingredient and, optionally, additional components whereby the pharmaceutical composition is a therapeutic or prophylactic vaccine. Finally, the present invention relates to the use of the polypeptide or a nucleic acid sequence encoding the same according to the present invention comprising IL-6 and a soluble IL-6 receptor for activation of effector and memory T-cells in a subject, in particular, in a human. Particularly, the T-cells are cytotoxic T-cells, CD8⁺ T-cells.

### Prior art

The induction of T cell immunity requires innate immune activation that generates inflammation and leads to maturation of professional antigen presenting cells (APCs), such as dendritic cells (DCs). Such matured APCs cross-prime naive CD8+ T cells and elicit differentiation into cytotoxic T lymphocytes (CTLs). Ideally, naive T cells receive membrane-associated and soluble costimulatory signals through CD28 together with receptors for IL-12 and type I IFN in addition to T cell receptor (TCR) stimulation. Such combination of signals is necessary to achieve optimal stimulation to induce sustained T cell proliferation and acquisition of T cell effector function. This differentiation process takes several days and is governed by a complex network of transcriptional regulators that control cell proliferation, effector function and survival (Kaech, S.M., and Cui, W. (2012). Nat Rev Immunol 12, 749-761). Effector function of CTLs is accomplished by secretion of anti-infectious cytokines such as TNF and IFNγ together with expression of death-inducing molecules such as Granzyme B (GzmB) or Perforin that are crucial for elimination of infected cells. Key to CTL differentiation and effector cell function are the T-box transcription factors T-bet and Eomesodermin (Eomes). In the absence of T-box transcription factors T cells fail to correctly differentiate into functional CTLs.

Various strategies are known for vaccination of individuals. Typically, a vaccination strategy is based on repetitive administration of the vaccine to the individual. By consecutive administration of the vaccine, the immune system should elicit an immune response against the antigen or pathogen to be vaccinated against. Various strategies exist. For example, common strategies are based on administration of antigenic components at predetermined time points. Typically, the antigenic components are administered together with adjuvants for enhancing the immune response in the individual. Vaccination may be conducted either therapeutically or prophylactically. While prophylactic vaccination strategies aim to stimulate the individual's immune system in developing preventive adaptive immunity to a pathogen, the goal of therapeutic vaccination strategy is to combat persisting infections or diseases, like cancer or chronic infections, present in the individuals body.

Vaccination should allow immunisation of said individual by developing immunological memory and protective immunity against the immunogen (antigen) present in the vaccine.

Typically, the immunisation enables the development of memory B cells and memory T cells being responsible for a swift response during a second encounter with the antigenic structure or immunogen.

Various vaccine strategies are adapted to allow the development of suitable memory B cell responses as well as memory T cell responses, namely, vaccination strategies to strengthen the antibody-based immune response, i.e. the humoral immune response. These vaccination strategies have been developed to allow prophylactic vaccination.

Despite the fact that vaccines have traditional been used for the prophylaxis of infectious diseases, recent findings suggested they are also powerful tools for the immunotherapy of transmissible diseases like viral infections or bacterial infections. In addition, vaccines can be used for the immune therapy or immune prophylaxis of autoimmune diseases, inflammatory diseases, tumors, allergies and for the control of fertility in human and/or animal populations.

The use of optimal adjuvants plays a crucial rule in vaccination. Antigens administered without adjuvant only rarely mediate an adequate immune response. In addition, not only the strength but also the quality of the elicited immune response matters. Stimulation of an incorrect immunization pattern may lead to immune pathological reactions and exacerbation of the symptoms of infection. In this context, the adjuvant can help to assist the desired immune response. In other words, an adjuvant can modulate the immune response or redirect the immune response to balance the immune response in the desired direction.

Typically, an adjuvant is a pharmacological and/or immunological agent that modifies the effect of other agents. Adjuvants may be added to vaccine to modify the immune response by boosting it such as to give a higher amount of antibodies or the humoral or cellular response in general and a longer lasting protection, thus, minimizing the amount of injected foreign material.

Typically, adjuvants are referred to compounds having immune potentiating properties, in particular, when co-administered with antigens and, typically, to enhance the humoral and/or cell mediated immune response. Various types of adjuvants are described in the art including inorganic compounds like alum or aluminium hydroxide, mineral oil, bacterial products, delivery systems including detergents but also combinations including Freund's complete adjuvant as well as cytokines. The goal of using adjuvants is to stimulate the immune system response to the target antigen but do not themselves confer specific immunities. Typically, vaccine strategies include administration of the antigen in combination with adjuvants, thus, leading to an induction of local inflammatory reaction resulting in an activation of antigen presenting cells which results in the activation of B-cells and T-cells accordingly.

Adjuvants described in the art so far typically induce humoral response in the individual. The known relevant mechanisms for activation of T-cells are based on co-stimulatory molecules induced on/or secreted by antigen presenting cells.

*In vivo,* priming of naive T cells by matured APCs occurs in lymphoid tissues such as lymph nodes and spleen, which facilitates the encounter of antigen-loaded APCs and naive T cells in highly specialized compartments, i.e. the T cell zones. Alternatively, naive CD8+ T cells are also stimulated outside of lymphoid tissues in peripheral organs such as the liver. Here, a highly abundant population of liver-resident cells, i.e. liver sinusoidal endothelial cells (LSECs) functions as scavenger cells to clear antigens from the circulation and as APCs to cross-present those antigens to circulating CD8+ T cells (von Oppen, N., et al., (2009). Hepatology 1664-1672). Naive T cell stimulation in the liver occurs without the requirement for innate immune stimulation and in the absence of conventional costimulatory signals. Such T cell stimulation by antigen-presenting LSECs results over a period of several days in the generation of memory T cells thus complementing conventional memory T cell generation induced during inflammation. These memory T cells generated by antigen-presenting LSECs over a period of several days did not show any direct cytotoxic effector function and similar to conventional memory T cells required combinatorial stimulation through costimulatory receptors for re-activation (Bottcher, J.P., et al. (2013). Cell Reports 3, 779-795).

The liver is an organ with unique immune functions that are determined by its particular microenvironment and its organ-resident antigen-presenting cells. Because of this distinct regulation of T cell responses the liver is considered to be a lymphoid organ. Although the immune system is in principle capable of clearing infections of the liver caused by viruses, bacteria or parasites, certain infections with hepatotropic pathogens such as Hepatitis B Virus (HBV) or Hepatitis C Virus or malaria parasites can persist and establish a chronic infection, which affects hundreds of millions of people worldwide. Several molecular mechanisms limiting the function and expansion of cytotoxic CD8 + T cells (CTLs) have been reported for the liver. These regulatory cues limit CTL function in the liver, which may serve to protect the infected liver from overwhelming immunopathology by inducing an oscillatory CTL effector function (Isogawa, M., et al., Immunity 23, 53-63 (2005)) but may also result in functional exhaustion or clonal elimination of pathogen-specific CTLs (Das, A. et al., J Exp. Med 205, 2111-2124 (2008). However, it was shown that large numbers of parasite-specific CTLs were able to eradicate infected hepatocytes indicating that the number of CTLs required to find and eliminate infected hepatocytes within the maze of liver sinusoids is critical for successful immune control of infection. Thus, the generation of large numbers of CTLs seems to be an important element for overcoming chronic infection. At present, no convincing immunotherapy for treatment of chronic viral infection of the liver or for treatment of intracellular pathogens in the liver or other pathogens of an individual as well as strategies for combating persistent tumors in an individual exists. Although adjuvant based vaccination strategies exist for generation of cellular immune responses, successful expansion of CTLs and eradication of the targets, like intracellular pathogens or tumors are not described in the art.

The generation of sufficient numbers of effector T-cells including CTLs for defence against pathogens as well as for combating persisting tumors is regulated in secondary lymphatic tissues by the extent of antigen presentation through appropriately matured dendritic cells. In liver, the liver sinusoidal cells, LSEC, represents APCs useful for activating effector T-cells including CTL.

Interleukin (IL) 6 is a cytokine with pleiotropic functions that contributes to anti-infectious immunity. IL-6 signals through the IL-6 receptor (IL-6R) and β subunit glycoprotein 130 (gp130). The IL-6 receptor exists as a membrane anchored protein (classic signaling) as well as in a soluble form (trans-signaling) that can be detected at sites of inflammation. Because of the restricted expression of IL-6R, many of the biological activities of IL-6 are attributed not to the cytokine alone but to the action of a soluble complex of IL-6 and IL6R, which initiates IL-6 trans-signaling by binding to ubiquitously expressed gp130 (Jones, S.A., Scheller, J., and Rose-John, S. (2011). J Clin Invest 121, 3375-3383). IL-6 has been shown to induce expression of acute phase proteins, to regulate development of DCs, to contribute to T helper 17 cell differentiation, to foster B cell development and antibody responses. A contribution of IL-6 signaling to development of CD8+ T cell immunity beyond induction of inflammation has been suggested (MacLeod, M.K., et al., (2011). Proc Natl Acad Sci U S A 108, 7914-7919) but not been characterized in detail.

Hence, there is still a need in the prior art to provide new compounds useful as adjuvants, particularly as adjuvants for the induction of effector T-cell responses allowing an improved and more rapid generation of effector T-cells as well as an improved proliferation of other stimuli.

Thus, the object of the present invention is the provision of new adjuvants which can elicit and/or enhance and/or modulate immune response in an individual or subject. In particular, the invention is based on the object of providing new adjuvants which act as a T-cell adjuvant, namely, allow to activate and transform T-cells into effector T-cells, in particular, cytotoxic T-cells.

### Brief description of the present invention

This technical problem is solved by the provision of the embodiments as characterized in the claims.

The present invention is in general concerned with a polypeptide complex comprising IL-6 and a soluble IL-6 receptor (sIL-6R) or a nucleic acid sequence encoding the same for use as an adjuvant. Said polypeptide complex or a nucleic acid sequence encoding the same according to the present invention for use as an adjuvant improves antigen specific immune responses in an individual or subject.

The present inventors found that a polypeptide complex comprising IL-6 and a soluble IL-6 receptor or a nucleic acid sequence encoding the same, in particular, Hyper-IL-6, are useful as adjuvants for activation of effector and memory T-cells, in particular, in humans.

In a further aspect, the present invention relates to a polypeptide complex wherein the polypeptide complex is Hyper-IL-6 or a nucleic acid sequence encoding the same. In addition, the present invention relates to pharmaceutical compositions comprising an adjuvant according to the present invention, namely, a polypeptide complex comprising IL-6 and a soluble IL-6 receptor or a nucleic acid sequence encoding the same as an adjuvant, a pharmaceutical active ingredient and, optionally, further components including pharmaceutically acceptable carrier, diluent, preservative, a further adjuvant other than the adjuvant according to the present invention, immunomodulator and/or excipient. The pharmaceutical composition is a therapeutic or prophylactic vaccine.

In a further aspect, the present invention relates to the use of the polypeptide complex or a nucleic acid sequence encoding the same according to the present invention for activation of effector and memory T-cells in a subject.

### Brief description of the drawings

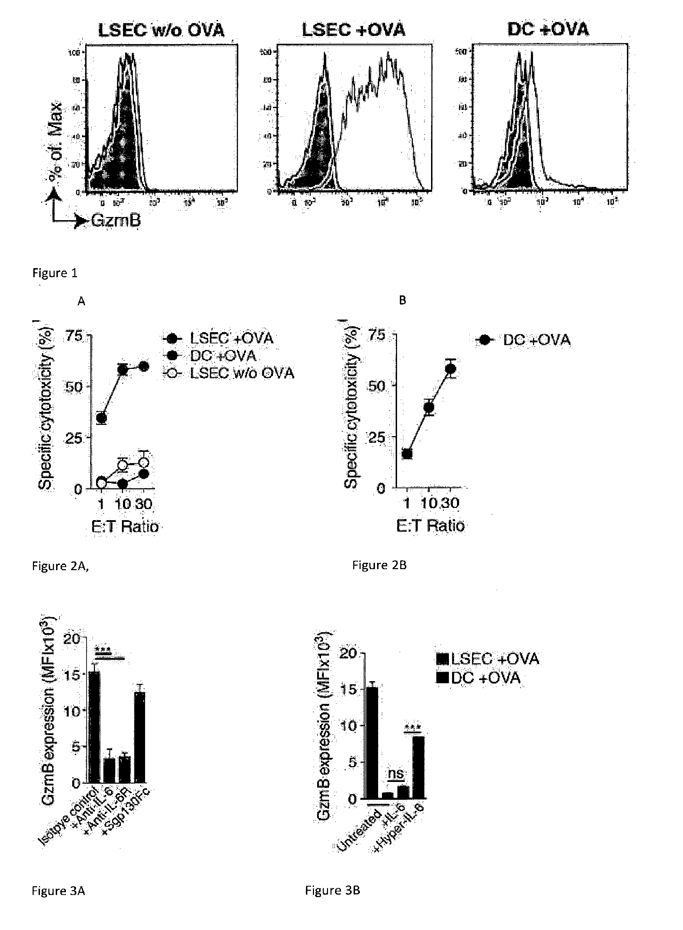

**Figure 1****. CD8⁺ T cell stimulation by cross-presenting LSECs but not DCs leads to rapid GzmB induction.** Analysis of naive OT-I T cell activation 18h after *in vitro* coculture with OVA-cross-presenting LSECs or matured DCs. T cells cultured on LSECs without OVA served as control. Intracellular GzmB protein is shown for the different types of cocultivation.
**Figure 2****. Rapid acquisition of effector functions in LSEC-stimulated T cells. (A, B)** Antigen-specific T cell cytotoxicity after **(A)** 18h or **(B)** 72h of co-culture with cross-presenting LSECs or DCs. data pooled from 3-4 experiments is shown, error bars represent mean ± SEM.

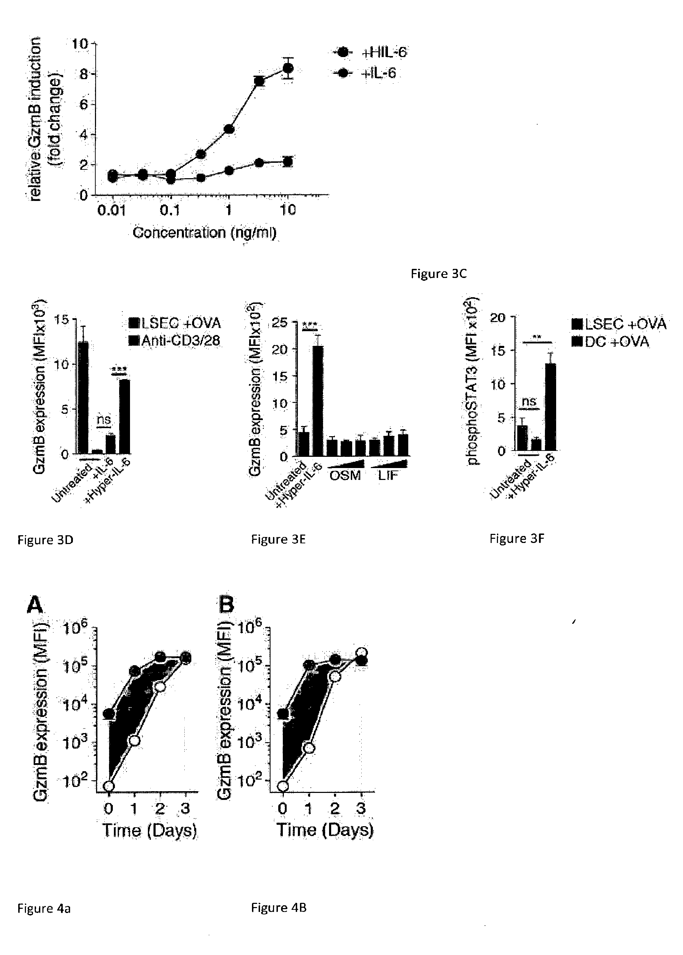

**Figure 3****. Rapid GzmB induction in T cells is mediated by IL-6 trans-signaling. (A)** Impact of neutralizing antibodies blocking IL-6 or IL-6R-signaling on rapid GzmB induction in LSEC-stimulated T cells. **(B-D)** GzmB expression in T cells 18h after stimulation by **(B)** anti-CD3/28 microbeads supplemented with exogenous IL-6 or Hyper-IL-6. For **(B)**, IL-6 and Hyper-IL-6 were used at 5 ng/ml, Sgp130Fc was used at 10µg/ml. Data of one of three independent experiments is shown as mean ± SD. **(E)** Analysis of GzmB expression in OT-I T cells 18h after stimulation by CD3/28 microbeads supplemented with the gp130-ligands Oncostatin M (OSM) and Leukemia inhibitory factor (LIF), each at 1-100ng/ml. Stimulation with CD3/CD28 beads plus 5ng/ml Hyper-IL-6 served as positive control. Data is representative of two independent experiments is shown as mean ± SD. **(F)** Intracellular expression of phosphoSTAT3 18 hours after stimulation of naive OT-I T cells with OVA-loaded LSEC, OVA-loaded DC or OVA-loaded DC supplemented with 5ng/ml Hyper-IL-6. Data is representative of two independent experiments and is shown as mean ± SD.

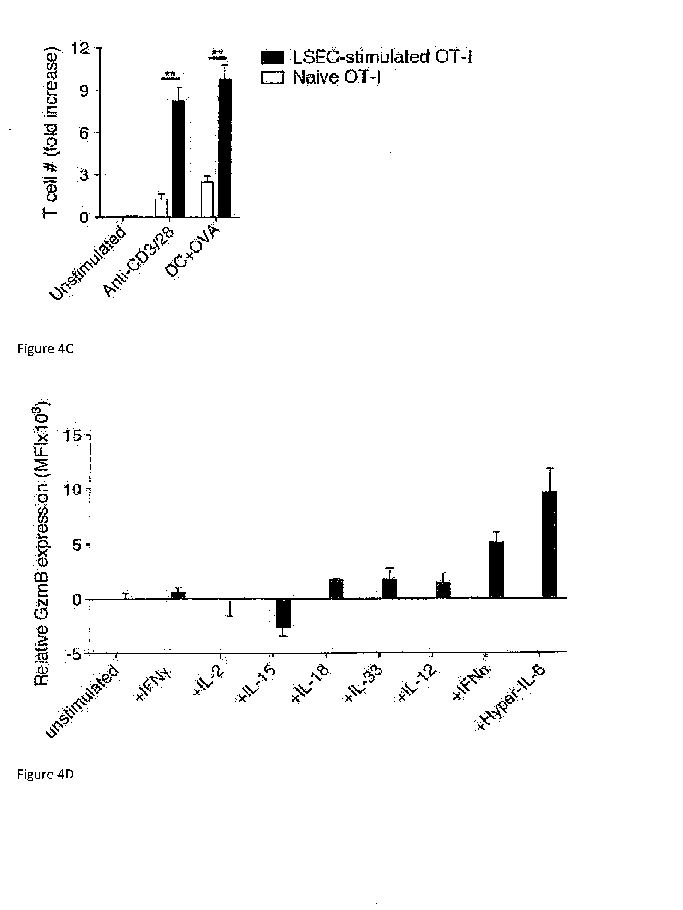

**Figure 4****. LSEC-stimulated GzmB-expressing T cells are responsive towards further stimulation.** Naive T cells or LSEC-stimulated GzmB-expressing T cells (after 18h) were re-stimulated with **(A)** cross-presenting matured DCs or **(B)** anti-CD3/28 microbeads for 3 days and analyzed for **(A, B)** GzmB expression after re-stimulation by PMA/lonomycin. **(C)** Expansion (fold increase) of naïve or LSEC-stimulated GzmB-expressing T cells after 3 days of stimulation with anti-CD3/28 microbeads or cross-presenting matured DCs. Increase in **(D)** GzmB production in naïve or LSEC-stimulated GzmB-expressing T cells after 8h incubation with inflammatory cytokines. The increase in MFI compared to un-stimulated T cells is shown. Data of one of three independent experiments is shown, error bars depict mean ± SD.

### Detailed description of the present invention

In a first aspect, the present invention relates to a polypeptide complex comprising IL-6 and a soluble IL-6 receptor or a nucleic acid sequence encoding the same for use as an adjuvant. The polypeptide complex or a nucleic acid sequence encoding the same according to the present invention allows to improve antigen specific immune responses in a subject.

In this connection, the term "adjuvant" means substances which are added and/or co-formulated in an immunization to an active antigen, i.e. the substance which provokes the desired immune response, in order to enhance or modulate the immune response against the active antigen.

As used herein, the term "individual" or "subject" which are used herein interchangeably refers to an individual or a subject in need of a therapy or prophylaxis. Preferably, the subject or the individual is a vertebrate preferred a mammal, particularly preferred a human.

As used herein, the term "carrier" refers to a diluent, excipient or vehicle.

As used herein, the terms "comprising", "comprises" and "comprised of" are synonymous with "including", "includes" or "containing", "contains" and are inclusive or open ended and do not exclude additional, non-recited members, elements or method steps. It will be appreciated that the terms "comprising", "comprises" and "comprised of" as well as "including", "includes" or "containing", "contains" as used herein comprised the terms "consisting of", "consists" and "consist of".

All references cited in the present specification are hereby incorporated by reference in their entirety. In particular, the teachings of all references herein specifically referred to are incorporated by reference.

Unless otherwise indicated, all terms used in disclosing the invention, including technical and scientific terms, have the meaning as commonly understood by one of the ordinary skilled in the art to which this invention belongs. By means of further guidance, term definitions are included to better appreciate the teaching of the present invention.

As used herein, the similar forms "a", "an" and "the" include both singular and plural references unless the context clearly dictates otherwise.

The terms "protein" and "polypeptide" are used interchangeably herein and refer to any peptide-bond-linked chain of amino acids, regardless of length or post translational modulation. Proteins useable in the present invention can be further modified by chemical modification. This means such a chemically modified polypeptide comprises other chemical groups than the 20 naturally occurring amino acids. Examples of such other chemical groups include without limitation glycosylated amino acids and phosphorylated amino acids. Chemical modifications of a polypeptide may provide advantageous properties as compared to the parent polypeptide, e.g. one or more of enhanced stability, increased biological half-life or increased water solubility. Chemical modifications applicable to the variants useable in the present invention include without limitation. PEGylation, glycosylation of non-glycosylated polypeptides etc.

"Pharmaceutically acceptable" means approved by regulatory agency of the federal or a state government or listed in the U.S. pharmacopeia or other generally recognized pharmacopeia for use in animals, and more particular, in humans.

The term "composition" is intended to include the formulation of the active compound with the adjuvant according to the present invention. The term "active ingredient" refers to the substance in a pharmaceutical composition or formulation that is biological active, i.e. provides pharmaceutical value, or a precursor thereof or a nucleic acid sequence encoding the same. In the context of the present invention, the active ingredient is a molecule that elicits an immune response.

As used herein, "prevent", "preventing", "prevention" or "prophylaxis" means preventing that a specific disease, disorder or condition occurs in a subject.

As used herein, "therapeutic" refers to treat a subject suffering from a disorder, disease or condition, thus, intended to produce a beneficial change in the condition of the individual.

The polypeptide or a nucleic acid sequence encoding the same according to the present invention is particularly for use as an adjuvant for therapeutic or prophylactic vaccination.

The polypeptide or a nucleic acid sequence encoding the same is useful a mucosal adjuvant, in particular, for intranasal, intraNALT, oral, intra-rectal, intrapulmonary, intrabronchial, intrarectal, conjunctival, intra-vaginal or intra-urethral administration, administration into the milk duct of the breast or by inhalation.

Alternatively, the polypeptide or a nucleic acid sequence encoding the same is for use as a systemic adjuvant, in particular, in subcutaneous, intravenous, intradermal or intramuscular administration.

The inventors recognized that the polypeptide complex or a nucleic acid sequence encoding the same according to the present invention acts directly on T-cells, thus, the polypeptide complex according to the present invention represents the first direct acting T-cell adjuvant. The term "direct acting" refers to the direct activation of T-cells independent of induction of inflammation and stimulation of antigen-presenting cells.

It is demonstrated herein that the polypeptide complex or a nucleic acid sequence encoding the same according to the present invention activates T-cells directly and, thus, in combination with additional stimuli, enhances proliferation of said T-cells. In addition, an improved and more rapid generation of effector T-cells in particular, cytotoxic T-cells (CTL) is possible.

The polypeptide complex or a nucleic acid sequence encoding the same according to the present invention is useful in the prophylactic or therapeutic vaccination against infection with an intracellular pathogen or in an anti-cancer vaccine. In an embodiment of the present invention, the intracellular pathogen is a viral pathogen, intracellular bacteria pathogen or an intracellular parasite.

In a further embodiment of the present invention, the polypeptide complex or a nucleic acid sequence encoding the same is for use in a prophylactic or therapeutic vaccine against a chronic or acute infection, in particular, chronic or acute viral infection.

The polypeptide complex or a nucleic acid sequence encoding the same according to the present invention is in an embodiment a polypeptide complex wherein the IL-6 and/or the soluble IL-6 receptor are derived from human IL-6 and/or human soluble IL-6 receptor, in particular, human IL-6 comprising SEQ ID No. 3 and/or human soluble IL-6 receptor comprising SEQ ID No. 4.

The structure of Hyper-IL-6 and the amino acid sequence thereof is disclosed e.g. in DE 196 08 813, see also Seq. ID No.2 or the nucleic acid sequence encoding the same of Seq. ID No. 1. The term "polypeptide complex comprising IL-6 and a soluble IL-6 receptor" as used herein refers to a polypeptide complex comprising the biological active part of IL-6 as well as the soluble IL-6 receptor or the part thereof responsible for binding its ligand, namely IL-6. The polypeptide complex refers both to a bimolecular polypeptide complex which features both the IL-6 polypeptide and the soluble IL-6 receptor polypeptide, and to a unimolecular polypeptide which includes IL-6 polypeptide and soluble IL-6 receptor polypeptide, at any order. Preferably, the bioactive portions of the soluble IL-6 receptor (sIL-6R) and IL-6 are connected to each other directly or via a flexible linker substantially as described in, e.g. WO 97/32891, WO 99/62253, WO 03/02981, as well as any biologically active analog or fragment thereof. The accessions number for IL-6 is M14584 (GenBank protein sequences data base), and for the soluble IL-6 receptors is M57230, and X12830.

The term "biologically active analog" refers to any homologous polypeptide to IL-6 or sIL-6R, which includes any amino acid substitution, deletion, or addition, while retaining the biological activity of the original polypeptide. Thus, a polypeptide complex comprising an analog or fragment of IL-6 and/or an analog or fragment of sIL-6R should retain the capability to directly activate the membrane receptor for the IL-6/sIL-6R polypeptide complex known as gp130 or at least one component of the downstream signing cascade of gp130.

The term "linker" relates to linkers of any kind, which are suitable for the binding of IL-6 with sIL-6R, examples of such linkers include, but are not limited to, bifunctional, chemical cross linkers, a disulfide-bridge connecting a first amino acid of IL-6 and a second amino acid of sIL-6R, a peptide or a polypeptide. An example of a suitable linker is the peptide of Seq. ID. No.5.

The unimolecular protein can be a fusion polypeptide. Thus, polypeptides featuring the bioactive portions of IL-6 and sIL-6R can be fused with each other and the linker can be a disulfide-bridge produced by the two polypeptides. In another embodiment, the liner is a peptide, which connects the two other polypeptides with each other. In a preferred embodiment, the polypeptide complex can include the human IL-6 polypeptide of SEQ ID No. 3 and/or the human soluble IL-6 receptor comprising SEQ ID No. 4 The peptide linker may have the amino acid sequence as set forth in SEQ ID No. 5.

The term "Hyper-IL-6" refers to a unimolecular protein as set forth in SEQ ID No. 2, or the nucleic acid sequence encoding the same of Seq. ID No. 1, consisting of amino acid residues 1 to 323 of human sIL-6R as set forth in SEQ ID No. 3 and amino acid residues 29 to 212 of human IL-6 as set forth in SEQ ID No. 4, connected with a flexible peptide linker as set forth and SEQ ID No. 5, substantially as described in WO 97/32891.

The amino acid substitutions may be silent substitutions or conservative or non-conservative substitutions. The skilled person is well aware of suitable substitutions possible.

In a preferred embodiment, the polypeptide complex is Hyper-IL-6 of SEQ ID No. 2. In another embodiment of the present invention, the polypeptide complex is for use as a direct active T-cell adjuvant.

In another embodiment, the nucleic acid sequence encoding the polypeptide complex according to the present invention is used. For example, the nucleic acid sequence is introduced by known means into the cells, e.g. in form of a vector. It may be possible that the antigenic compound is introduced as nucleic acid sequence as well, e.g. in the same vector system.

In another embodiment, the present invention relates to a pharmaceutical composition comprising the polypeptide complex according to the present invention as an adjuvant, a pharmaceutical active ingredient and, optionally, a pharmaceutically acceptable carrier, diluent, preservative, a further adjuvant other than the compounds according to the present invention, immune modulator and/or excipient, in particular, whereby the pharmaceutical composition is a therapeutic or prophylactic vaccine.

Thus, in a further aspect, the present invention relates to pharmaceutical compositions comprising compounds as defined herein as adjuvants, together with a pharmaceutically active ingredient, and, optionally, a pharmaceutically acceptable carrier. The pharmaceutical composition may be administered with a physiologically acceptable carrier to a patient, as described herein. The term "carrier" refers to a diluent, adjuvant other than the adjuvant according to the present invention, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Saline solutions and aqueous dextrose and glycerol solutions can also be employed as liquid carriers, particularly for injectable solutions. Suitable pharmaceutical excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium, carbonate, etc. Examples of suitable pharmaceutical carriers are described in "Remington's Pharmaceutical Sciences" by E.W. Martin (18th ed., Mack Publishing Co., Easton, PA (1990)). Such compositions will contain a therapeutically effective amount of the active ingredient, typically the antigenic compound, together with a suitable amount of carrier so as to provide the form for proper administration to the patient. The formulation should suit the mode of administration.

Typically, pharmaceutically or therapeutically acceptable carrier is a carrier medium which does not interfere with the effectiveness of the biological activity of the active ingredients and which is not toxic to the host or patient. The pharmaceutical composition for use in connection with the invention can be formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxides, isopropylamine, triethylamine, 2-ethylamino ethanol, histidine, procaine, etc. "Therapeutically- or pharmaceutically-effective amount" as applied to the compositions of the instant invention refers to the amount of composition sufficient to induce a desired biological result. That result can be alleviation of the signs, symptoms, or causes of a disease, or any other desired alteration of a biological system. In the present invention, the result will typically involve an increase in the immunological responses to infection or a suppression of the responses to inflammatory processes or the treatment or prophylaxis of cancer etc.

In vitro assays may optionally be employed to help identifying optimal dosage ranges. The precise dose to be employed in the formulation will also depend on the route of administration, and the seriousness of the disease or disorder, and should be decided according to the judgment of the practitioner and each patient's circumstances. Effective doses may be extrapolated from dose-response curves derived from in vitro or animal model test systems. Preferably, the pharmaceutical composition is administered directly or in combination with an adjuvant.

The term "administered" means administration of a therapeutically effective dose of the aforementioned pharmaceutical composition comprising the adjuvants together with the active ingredient as defined herein to an individual. By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

In still another embodiment, the present invention relates to methods of treating individuals suffering from diseases, disorders or conditions as identified herein comprising the step of administering to said individual an effective amount of a pharmaceutical comprising an active ingredient, the polypeptide complex or a nucleic acid sequence encoding the same as an adjuvant according to the present invention, and, optionally, a pharmaceutically acceptable carrier. In particular, the method is useful for preventing or treating cancer and/or tumours, such as, melanoma, prostate, breast, colorectal, stomach, throat and neck, pancreatic, cervical, ovarian, bone, leukemia and lung cancer; viral infections, such as, hepatitis B, hepatitis C, human immunodeficiency virus, helico-bacter pylori, herpes virus, etc.; bacterial infections, such as tuberculosis, leprosy and listeriosis, and parasitic infections such as malaria.

Further, the pharmaceutical composition may contain additionally components, e.g. compounds like one or more anti-inflammatory molecules, anti-angiogenic molecules, cytotoxic molecules, immunomodulatory molecules, preferably chemokines, cytokines, CD40 ligand, costimulatory molecules or antibodies or mixtures thereof.

In addition, the pharmaceutical composition described herein may be characterized in that the components of the pharmaceutical composition are associated and/or incorporated and/or coated to a physical particle, preferably microparticle, nanoparticle, liposome, ISCOM, copolymer and/or biological particle, preferably bacterial ghosts.

The methods are applicable to both human therapy and veterinary applications. The compounds described herein having the desired therapeutic activity may be administered in a physiologically acceptable carrier to a patient, as described herein. Depending upon the manner of introduction, the compounds may be formulated in a variety of ways as discussed below. The concentration of therapeutically active compound in the formulation may vary from about 0.1-100 wt%. The agents may be administered alone or in combination with other treatments.

The pharmaceutical composition according to the present invention is for use as a vaccine wherein the active ingredient is an antigenic compound, in particular, an antigenic compound derived from an intracellular pathogen or cancer cell. It is particularly preferred that the intracellular pathogen is X3275-15 an intracellular viral pathogen, an intracellular bacterial pathogen or an intracellular parasite pathogen. For example, the viral pathogens are any hepatitis viruses including hepatitis B or C, persisting and liver. Examples for parasites include *Schistosoma spp* or *Plasmodia spp.*

That is, the polypeptide or a nucleic acid sequence encoding the same according to the present invention is particularly useful in a prophylactic or therapeutic vaccine against a chronic or acute infection, in particular, a chronic or acute viral infection, like chronic infections of the liver, e.g. by hepatitis B or by hepatitis C. Accordingly, the antigenic compound or a nucleic acid sequence encoding the same present in the pharmaceutical composition according to the present invention is an antigenic compound described for the intracellular pathogen or cancer cell, respectively. The skilled person is well aware of suitable antigenic compounds as described in the art. For example, the Hepatitis B virus core and surface antigens for vaccination against chronic Hepatitis B and the glypican 3 antigen for vaccination against hepatocellular carcinoma.

In an embodiment of the present invention, the pharmaceutical composition is in form of artificial particles, in particular, in form of nanobeads or microparticles. These artificial particles comprises the polypeptide according to the present invention in combination with molecules having agonistic activity on CD28, and peptide loaded MHC-molecules. That is, these artificial particles, e.g. in form of nanobeads or microparticles represents artificial antigenic presenting cells, allowing activation of antigen specific lymphocytes, in particular, antigen specific T-cells.

In case of artificial particles, these artificial particles may be administered by known application routes, in particular may be administered subcutaneously or intramuscularly, analogues to known administration routes.

Another embodiment of the present invention relates to a vector system containing a nucleic acid sequence encoding the polypeptide complex according to the present invention for use in altering an immune response, like, acting as an adjuvant. In an embodiment, the vector system may encode additionally the antigenic compound and/or further accessory immune stimulatory molecules, like CD80/CD86.

In another embodiment, administration of the polypeptide or a nucleic acid sequence encoding the same according to the present invention, e.g. in form of a pharmaceutical composition according to the present invention is possible using the antigen as active ingredient or a nucleic acid sequence encoding the same, adjuvants other than the polypeptide complex according to the present invention and the polypeptide complex or a nucleic acid sequence encoding the same according to the present invention, e.g. Hyper-IL-6. It is demonstrated by the inventors that the polypeptide complex, e.g. in form of IL-6, acts independently from the CD28 mediated signalling. In addition, it has been recognized by the inventors that a stronger T-cell activation occurs. Furthermore, small amounts of the polypeptide complex or a nucleic acid sequence encoding the same according to the present invention, e.g. of the Hyper-IL-6 are sufficient to achieve T-cell activation accordingly. Moreover, side effects are less compared to known methods, for example an overwhelming inflammation at the site of administration or the site of action.

The pharmaceutical composition according to the present invention is particularly useful for vaccination against chronic infection of the liver, e.g. of chronic hepatitis B, or chronic hepatitis C.

That is, the pharmaceutical composition according to the present invention is in a preferred embodiment a composition wherein the polypeptide complex is Hyper-IL-6, the compound having agonistic activity on CD28 is an agonistic CD28 antibody, and the peptide loaded MHC is a peptide loaded MHC wherein the peptide is an antigenic determined derived from an intracellular pathogen or cancer cell, like an intracellular pathogen of chronic or acute infection, in particular infection of the liver.

Finally, the present invention relates to a polypeptide complex or a nucleic acid sequence encoding the same according to the present invention for activation of effector and memory T-cells in a subject, in particular, in humans. The present invention relates in addition to a method for therapeutic or prophylactic vaccination including the step of administering the polypeptide complex or a nucleic acid sequence encoding the same according to the present invention as an adjuvant together with an antigenic compound or a nucleic acid sequence encoding the same whereby administration may be effected simultaneously, sacredly or sequentially.

In another embodiment, the method for prophylactic or therapeutic vaccination comprises the step of administering an artificial particle as described herein to an individual in need thereof.

Individuals in need of the pharmaceutical composition or the polypeptide complex according to the present invention are individuals suffering e.g. from infection with an intracellular pathogen or cancer. For example, the subjects in need thereof are subjects suffering from chronic infection e.g. chronic viral infection, for example infection of the liver. Alternatively, the subject in need thereof are subjects which are at risk of getting infected with a pathogen or being at risk of developing cancer.

These and other embodiments are disclosed and encompassed by the claims and the description examples of the present invention. Further literature concerning any one of the methods, uses and compounds to be employed in accordance with the present invention may be retrieved from public libraries.

### Examples

The examples are designed in order to further illustrate the present invention and serve a better understanding. They are not to be construed as limiting the scope of the invention in any way.

### Experimental procedures

**Mice.** B6.CH-2^{bm1}, CD80/86^{dko}, C57BL/6, CD90.1⁺ C57BL/6, T cell receptor transgenic OT-I and CD45.1⁺ OT-I were bred under specific pathogen-free (SPF) conditions in the central animal facility of the University Hospital Bonn. Chimeric animals were generated as described. Mice were kept under SPF-conditions and *in vivo* experiments were approved by the local animal care commission.

**Analysis of T cell priming *in vivo***. Splenic OT-I T cells were enriched by autoMACS using the untouched CD8 T cell isolation kit (Miltenyi) and CD8⁺CD45.1⁺CD44^{low} OT-I T cells were sorted by FACS. 1x10⁶ were injected i.v. into C57BL/6 wildtype mice or chimeric animals which received 1 mg endotoxin-free Ovalbumin (Hyglos). 18h later, lymphocytes from spleen, liver or lymph nodes were purified as described (Bottcher et al., 2013, see above) and the expression of GzmB was analyzed within CD3⁺CD8⁺CD45.1⁺ T cells. In some experiments, FACSsorted naive T cells were labeled with CFSE prior to adoptive transfer.

**Ba/F3 cell experiments.** Ba/F3 cells were stably transduced with human IL-6R cDNA, and Ba/F3-gp130 cells with eGFP cDNA as described (Ketteler, R., et al.,(2002). Gene therapy 9, 477-487). Expression and purification of IL-6, Hyper-IL-6 and sgp130Fc has been described previously (Fischer, M., et al., (1997). Nature biotechnology 15, 142-145). For the co-culture experiment, 5x10⁴ cells were cultivated at the indicated ratios for two days. Cell viability was determined with Cell Titer Blue Cell viability assay reagent (Promega) following the manufacturer's protocol.

**Cell isolation and coculture experiments.** Dendritic cells or naive OT-I T cells used in *in vitro* coculture experiments were isolated from spleen and purified by autoMACS (Miltenyi Biotec). LSEC were isolated by gradient centrifugation followed by immunomagnetic sorting (CD146) (Diehl, L., et al., (2008). Hepatology 47, 296-305). Cocultures of naive OT-I cells with LSEC or matured DCs were conducted as described previously (Bottcher et al., 2013, see above). LSECs or matured DCs were loaded with 100µg/ml Ovalbumin (OVA) and OT-I T cells were added to antigen-presenting cells in a ratio of 1:1. In some experiments, OT-I T cells were labeled with 1 µM CFSE (carboxyfluorescein diacetate succinimidyl ester; Invitrogen) for 15 min at 37°C prior to the coculture assay. Determination of cross-presentation was done by incubation of OVA-loaded APCs with H-2K^{b}-restricted OVA-specific B3Z cells and analysis of IL-2 release by ELISA after 20h. In some experiments, neutralizing antibodies for 41BBL (clone TKS-1), CD40L (MR1), CD70 (FR70), OX40L (RM134L), ICAM (YN1/1.7.4), PD-1 (J43), CD80 (16-10A1), CD86 (GL1), IL-6 (MP5-20F3), IL-6R (polyclonal, AF1830, R&D Systems), IFN-gamma (XMG1.2), IL-15 (A10.3), IL-2 (JES6-1A12), IL-12 (clone C17.8) or Type I IFN signaling (anti-mouse IFNAR1, clone MAR1-5A3) were added to cocultures at 10 µg/ml. In some experiments, stimulation of naïve T cells by matured DCs or anti-CD3/28 microbeads was done after addition of recombinant cytokines IL-2, IL-6; Hyper-IL-6 (HIL-6) or stimulating anti-CD28 antibody (10µg/ml, ebioscience).

**Quantification of *gzmB* gene expression by real-time PCR.** Total mRNA was isolated using the RNeasy Micro Kit (Qiagen; including DNase digestion) and transcribed reversely into cDNA using the AffinityScript™ Multiple Temperature Reverse Transcriptase (Stratagene). Real-time PCR was performed using the absolute QPCR SYBR Green PCR Mix (Abgene) in combination with specific primers (QuantiTect Primer Assay; Qiagen) for murine GzmB and GAPDH. GAPDH was used as reference gene. All real-time PCR reactions were performed in a C1000™ Thermal Cycler (Bio-Rad).

**Flow cytometry and fluorescence activated cell sorting (FACS).** Flow cytometric analyses and assessment of mean fluorescence intensity (MFI) were conducted with a LSR Fortessa or Canto II (BD Biosciences). Data were analyzed using FlowJo software (Tree Star, Ashland, OR). LIVE/DEAD Fixable Violet or Near-IR Dead Cell Stain kit (Invitrogen) was used to exclude dead cells in all experiments, anti-CD16/32 antibody (2.4G2) was used to block unspecific antibody binding via Fc receptors. Antibodies were purchased from Biolegend or ebioscience. The following antibodies were used: CD3 (17A2), CD8□ (clone 53-6.7), CD25 (3C7), CD27 (LG.7F9), CD44 (IM7), CD45.1 (A20), CD62L (MEL-14), CD69 (H1.2 F3), CD90.2 (HIS51), H-2K^{b} (5F1), Lamp1 (1DB4). For intracellular staining of cytokines, cells were fixed in 4% PFA and intracellular staining by anti-IFN gamma antibodies (XMG1.2) or anti-IL-2 antibodies (JES6-5H4) was performed in Permeabilization Buffer (ebioscience) for 30 minutes. Staining of T-bet (eBio4B10), Eomes (Dan11mag) or Granzyme B (anti-human, cross-reactive with mouse, clone GB11) was performed using the Foxp3/Transcription factor staining buffer set from ebioscience. Quantification of total T cell numbers was done with fluorochrome-labeled microbeads (CountBright absolute counting beads, life technologies, Invitrogen). Fluorescence activated cell sorting (FACS) of naive T cells was performed with a DIVA cell sorter (BD). Expression of phosphoSTAT3 was determined by flow cytometry after intracellular staining with anti-STAT3pY705 (clone4/P-STAT3) using the phosflow kit from BD. Staining with the corresponding isotype antibody served as control.

**Analysis of T cell effector functions.** Analysis of GzmB expression was performed directly after isolation of T cells *ex vivo* or from *in vitro* cocultures without further stimulation. T cell coculture with anti-CD3/CD28 microbeads (Invitrogen) was done in presence of recombinant mouse IL-2 and IL-12 (5 ng/ml). In order to determine the potential of T cells to produce effector cytokines (see Fig. 4E and F), T cells were stimulated with PMA (5ng/ml; Sigma Aldrich) and lonomycin (200 ng/ml, Sigma Aldrich). To analyze T cell proliferation, naive CD8⁺ T cells were labeled with 1 µM CFSE (Invitrogen) before coculture experiments or adoptive transfer, and CFSE-dilution was measured by flow cytometry. Analysis of cytokine-mediated T cell activation was done by incubating purified T cells for 8h at 37°C with cytokines IL-2, IL-15, IL-18, IL-33, IFN-gamma, TNF (all 10ng/ml), IL-12 (5ng/ml), Hyper-IL-6 (5ng/ml) or IFN-alpha (Type 4, 1000 U/ml) on CD90.1⁺ splenocytes as feeder cells. Brefeldin A and Monensin were added during the last 2h of stimulation. Expression of GzmB and IFN gamma was subsequently analyzed within CD90.2⁺ OT-I T cells by flow cytometry. Determination of antigen-specific specific cytotoxicity was determined *in vitro* (Diehl et al., 2008, see above). LPS-free Ovalbumin (Hyglos) was used for *in vivo* experiments at a concentration of 1 mg/mouse.

**Statistical analysis.** Data was compared using one-way analysis of variance (ANOVA) or the unpaired two-tailed Student's t-test. Data are shown as mean ± standard error of the mean (SEM) or mean ± standard deviation (SD) with *p<0.05, **<0.01, ***p<0.001.

### Results

### Naive CD8⁺ T cells rapidly express Granzyme B after antigen-presentation by LSECs

LSECs are potent antigen (cross)-presenting cells resident to the liver. Whereas cross-priming by DCs requires several days for cytotoxic effector T cell differentiation, cross-presentation by LSEC to naive CD8⁺ T cells leads to an accelerated generation of memory T cells (Bottcher et al., 2013, see above). Here, we investigate the early phase, i.e. the first 24 hours of the interaction between cross-presenting LSEC and naive CD8⁺ T cells compared to DCs. Coculture of naive H-2K^{b}-restricted OVA-specific TCR-transgenic CD8⁺ T cells (OT-I) with LSECs cross-presenting OVA resulted in rapid T cell activation. After 24h of T cell contact with cross-presenting LSEC (LSEC-stimulated T cells) increased expression levels for CD25, CD44 and CD69 were observed compared to T cells cultured with LSEC without antigen. Such increased expression of activation markers was indistinguishable from that following activation by cross-presenting matured DCs for 24h (DC-stimulated T cells). Similarly, activation-induced reduction of CD62L expression was similar between LSEC- and DC-stimulated T cells. Although LSECs and DCs were equally efficient in cross-presentation and initial stimulation of naive OT-I T cells leading to expression of cytokines such as IL-2, TNF and Interferon γ during the first 12h, we found one fundamental difference when analyzing proteins important for T cell cytotoxic effector function. Stimulation by cross-presenting LSECs already within 18h led to strong upregulation of the serine protease Granzyme B (GzmB) (**Fig. 1**), that is key for cytolytic function of effector T cells. In contrast, cross-priming by matured DCs did not induce such rapid GzmB expression within 18h (**Fig. 1**). This finding was corroborated by the prominent upregulation of *gzmb* mRNA expression in LSEC-but not DC-stimulated T cells within 18h. Acquisition of GzmB expression and development of effector function upon classical T cell priming by DCs is only observed after T cells had proliferated. The rapid GzmB expression in LSEC-stimulated T cells, however, occurred before proliferation started, since no CFSE-dilution was observed in GzmB-expressing T cells, which suggests a distinct developmental process. Time kinetic analysis of *gzmb* gene expression between T cells stimulated by LSECs or DCs confirmed the rapid GzmB induction by LSECs but not DCs. These experiments further demonstrated that DC-stimulated T cells had sustained GzmB expression after 48h whereas *gzmb* levels in LSEC-stimulated T cells declined to baseline at this point. Taken together, these results indicate a difference between LSECs and mature DCs in the dynamics of GzmB induction, namely a rapid but transient expression induced by LSECs and a later but sustained expression induced by DCs. These two time points, i.e. 18h and 48h, are referred to here as early and late GzmB induction, respectively.

We next investigated whether antigen-presentation *in vivo* also led to rapid GzmB expression in T cells. We adoptively transferred FACSorted CD45.1 ⁺ naive CD44^{low}CD62L⁺ OT-I T cells into CD45.2⁺ recipients and challenged these mice with soluble endotoxin-free OVA. 18h later GzmB-positive OT-I T cells were only observed in liver. Consistent with their antigen-specific activation, GzmB-positive T cells also expressed activation markers such as CD44 and CD69. Not all transferred T cells isolated from the liver showed increased GzmB expression, which may relate to the fact that *in vivo* only some naive T cells engage in closer interaction with cross-presenting LSEC in hepatic sinusoids (von Oppen, et al., 2009, see above). Since we transferred only naive CFSE-labeled CD44^{low} T cells, we can exclude that rapid GzmB induction *in vivo* resulted from re-activation of CD44⁺ memory T cells. GzmB-positive T cells isolated from liver had not entered cell cycle as demonstrated by absence of CFSE-dilution similar to LSEC-stimulated GzmB-positive T cells *in vitro.* These results suggested that LSEC cross-presenting OVA *in vivo* could be responsible for rapid GzmB induction in naive OT-I T cells. To address this question, we used a chimeric mouse model where H-2K^{b} expression is restricted to non-myeloid cells (bm1->C57BL/6), which together with injection of endotoxin-free OVA restricts cross-presentation to liver-resident LSEC *in vivo* (Bottcher et al., 2013; von Oppen et al., 2009, see above). Transfer of FACS sorted naive OT-I T cells into (bm1->C57BL/6) chimeric mice followed by injection of endotoxin-free OVA resulted in GzmB induction in T cells isolated 18h later from liver that was indistinguishable from T cells stimulated in wild type mice with ubiquitous H-2K^{b} expression. No GzmB expression was observed 18h after T cell transfer into OVA-challenged (C57BL/6->bm1) chimeric mice, where only bone marrow-derived APCs but not LSECs cross-present circulating OVA. Taken together, these results support the notion that LSEC cross-presentation leads to a unique differentiation process in naive CD8⁺ T cells characterized by rapid GzmB induction within 18h.

### LSEC-stimulated GmzB-positive T cells gain effector functions

Analysis of GzmB expression suggested that naive T cells stimulated by cross-presenting LSEC rapidly gained cytotoxic effector function. Consistent with this assumption, TCR re-stimulation of LSEC-stimulated T cells *in vitro* resulted in localization of LAMP1 molecules to the cell surface, indicating that LSEC-stimulated T cells have the potential to secrete GzmB from intracellular stores. LSEC-stimulated GzmB-expressing T cells efficiently killed peptide-loaded target cells whereas unloaded control cells were not affected (**Fig. 2A**). The rapid development of antigen-specific cytotoxicity within 18h required cross-presentation because OT-I T cells co-cultured with LSEC in the absence of OVA did not kill target cells at all (**Fig. 2A**). In contrast, DC-stimulated T cells were not cytotoxic at 18h after T cell priming (**Fig. 2A**) but acquired cytotoxic function later (**Fig. 2B**). This suggests that development of effector T cell functions occurs via different means through cross-presenting LSECs compared to matured DCs.

Cytotoxic CD8⁺ T cells are known to also secrete effector cytokines such as IFNγ, IL-2 and TNF. LSEC-stimulated GzmB-positive T cells, despite their potent cytotoxic function did not produce significant amounts of IFNγ, IL-2 or TNF compared to DC-stimulated T cells, demonstrating that cytokine production does not correlate with rapid acquisition of cytotoxicity.

### IL-6 trans-signaling elicits early GzmB expression in T cells

We wondered which signals were responsible for LSEC-induced rapid expression of GzmB in T cells. We first investigated the contribution of TCR signaling strength. LSECs and DCs were equally efficient in cross-presentation over a wide OVA concentration range, i.e. delivery of signals through the TCR. However, augmenting antigen concentrations did not result in rapid GzmB induction in DC-stimulated T cells. Notwithstanding the direct correlation of TCR activation and GzmB-induction in LSEC-stimulated T cells, the results therefore indicate that the extent of TCR-signaling alone does not determine rapid GzmB upregulation in T cells. It rather suggested that LSECs provided a distinct signal to T cells for rapid GzmB-induction within 18h that is not provided by matured DCs.

We therefore analyzed the contribution of co-signaling molecules to rapid GzmB induction in LSEC-stimulated T cells. Even in the absence of CD28 signals in T cells stimulated by cross-presenting CD80/86^{dko} LSECs we observed a robust induction of rapid GzmB expression. Similarly, neutralizing antibodies blocking the interaction between receptor-ligand pairs relevant for T cell co-stimulation, such as 4-1BB-4-1BBL, CD40-CD40L, CD70-CD27 or OX40-OX40L, or ICAM-1 did not influence LSEC-induced rapid GzmB expression. Interestingly, blockade of the co-inhibitory receptor PD-1, that controls TCR-signaling, also did not increase GzmB-expression levels, strengthening again the notion that GzmB expression in LSEC-stimulated T cells was not regulated by the strength of TCR-signaling.

Next, we analyzed the contribution of soluble mediators that serve as co-signaling molecules for T cell activation. Blockade of IL-12 did not affect GzmB expression, consistent with lack of IL-12 expression by LSEC. Similarly, neutralization of type I IFN using anti-IFNAR antibodies did not modify GzmB expression levels in LSEC-stimulated T cells. To exclude redundant functions of these co-signaling molecules, we simultaneously blocked CD28, IL-12 and type I IFN signals. This concurrent blockade did not influence rapid LSEC-mediated GzmB-induction in T cells. Also blockade of other soluble mediators known to enhance T cell effector functions, i.e. IFNγ or IL-15, had no effect, but blocking IL-2 strongly reduced rapid GzmB induction in LSEC-stimulated T cells. Whereas CD28 signaling was dispensable for rapid GzmB-induction in LSEC-stimulated T cells it was crucial for late GzmB-induction in DC-stimulated T cells. Neutralization of IL-2 also impaired late GzmB-induction in DC-stimulated T cells. Supplementation of co-cultures of matured antigen-presenting DCs and T cells with exogenous IL-2 did not enable rapid GzmB-induction in T cells within 18h, which suggests that IL-2 is not sufficient for early GzmB induction but rather acts as a co-factor. Since we observed that LSEC and DC released large concentrations of IL-6 during cross-presentation to naïve T cells, we tested whether IL-6 signaling was involved in rapid GzmB-induction. IL-6 classic signaling requires presence of the IL-6 receptor (IL-6R) and the ubiquitously expressed signaling transducing unit gp130. Cells that do not express membrane-bound IL-6R can be activated through IL-6 trans-signaling, as the complex of IL-6/sIL-6R triggers gp130 activation (Jones et al., 2011, see above). Neutralization of IL-6 or blockade of the IL-6R both abrogated rapid GzmB-induction in LSEC-stimulated T cells (**Fig. 3A**), raising the question whether classical or IL-6 trans-signaling was involved. Addition of soluble gp130 (sgp130Fc) to co-cultures of cross-presenting LSEC and naive T cells did not abrogate rapid GzmB induction (**Fig. 3A**), although trans-signaling by soluble IL-6/IL-6R complexes is completely blocked by sgp130Fc (Jones et al., 2011). We therefore determined whether IL-6R expression on cells in trans could also lead to IL-6 signaling on an IL-6R-deficient cell population, such as CD8⁺ T cells (Jones et al., 2011). To address this issue, we used Ba/F3 cells stably transduced with either gp130 (Ba/F3-gp130) or IL-6R (Ba/F3-IL-6R). Whereas Ba/F3-gp130 cells only proliferated in response to Hyper-IL-6 but not IL-6 alone, addition of IL-6 to Ba/F3-gp130 cells co-cultured together with Ba/F3-IL-6R cells was sufficient to induce proliferation in Ba/F3-gp130 cells. Furthermore, such proliferation even occurred in the presence of sgp130Fc. These data indicate that not only soluble IL-6/IL-6R complex but also surface-bound IL-6/IL-6R was sufficient to induce IL-6 trans-signaling in IL-6R-deficient cells. If IL-6 trans-signaling was relevant for rapid GzmB-induction, then IL-6 coupled to its receptor (Hyper-IL-6) should lead to GzmB induction in T cells stimulated by antigen-presenting DCs. Indeed, addition of Hyper-IL-6 but not IL-6 alone triggered GzmB expression within 18h in DC-stimulated T cells (**Fig. 3B****,** **3C**). Addition of Hyper-IL-6 to anti-CD3/CD28-coated microbeads as artificial APCs also induced GzmB-expression within 18h (**Fig**. **3D**), which demonstrates that IL-6 trans-signaling acting on T cells was required and sufficient to drive rapid development of effector functions. Interestingly, other ligands for gp130 such as Oncostatin M (OSM) or Leukemia inhibitory factor (LIF) failed to induce GzmB expression (**Fig. 3E**). Furthermore, Hyper-IL-6 treatment induced STAT3 phosphorylation in T cells in combination with anti-CD3/CD28-coated microbeads within 18h whereas LSEC-stimulated T cells only showed a non-significant increase in STAT3-phosphorylation (**Fig. 3F**). These results indicate that Hyper-IL-6 initiates a so far undefined signaling cascade relevant for rapid effector T cell differentiation.

### Rapid acquisition of effector cell function attributes superior activation potential to T cells

Next, we asked whether the rapid but transient induction of effector T cell function by LSEC changed the responsiveness towards further stimulation. Whereas GzmB expression declined in LSEC-stimulated T cells over time, re-activation of these T cells 18h after their initial stimulation through LSEC by matured DCs or anti-CD3/CD28 microbeads led to a sustained and further increase in GzmB expression over several days (**Fig. 4A****,B**). During such treatment, naive T cells required more than 48h to reach similar levels of GzmB expression compared to LSEC-stimulated T cells (**Fig. 4A****,B**). Also IFNγ expression was increased upon such re-activation of LSEC-stimulated T cells. These findings were confirmed by challenge of T cells stimulated by LSEC for 18h or naive T cells with PMA/lonomycin followed by determination of cytokine production within 4h. Under these conditions, LSEC-stimulated T cells showed strong expression of IL-2 and IFNγ whereas naive T cells did not produce any cytokines, demonstrating that GzmB-expressing T cells are more responsive to re-activation than naïve T cells. Such increased IL-2 expression together with expression of its receptor CD25 suggested a capacity for rapid proliferation. Indeed, LSEC-stimulated T cells showed vigorous expansion within 72h of re-stimulation with antigen-presenting DCs or anti-CD3/CD28 microbeads that was more prominent than proliferation of naive T cells (**Fig. 4C**).

T cell effector function is not only triggered by antigen-specific stimulation but can also be evoked by cytokines released during inflammation in an antigen-independent fashion. We therefore determined whether LSEC-stimulated T cells were susceptible for such "innate activation" by inflammatory cytokines. Indeed, we observed a further upregulation of GzmB expression if LSEC-stimulated T cells were incubated with Type I IFN or Hyper-IL-6 in the absence of antigen (**Fig. 4D**) and a moderate upregulation of GzmB expression upon incubation with IFNγ, IL-12, IL-18 or IL-33, but no effect upon incubation with IL-2 or IL-15 (**Fig. 4D**). Importantly, naive OT-I T cells did not show any GzmB expression under these conditions (**Fig. 4D**). Likewise, both IL-12 and Type I IFN evoked production of the T cell effector cytokine IFNγ in LSEC-stimulated T cells but not in naive T cells. These results indicate that rapid induction of effector T cell functions by cross-presenting LSEC in the liver may provide an advantage to mount protective immunity against systemically circulating pathogens.

### Discussion

We discovered that IL-6 trans-signaling was required for LSEC-induced rapid GzmB induction in T cells and was also sufficient to establish rapid GzmB expression in T cells when co-administered together with cross-presenting DCs. LSEC produced IL-6 during cross-presentation and stimulation of naïve CD8⁺ T cells. IL-6 signaling is only initiated upon association with the IL-6R and binding to the ubiquitously expressed gp130. Since CD8⁺ T cells do not express IL-6R, trans-signaling through soluble Hyper-IL-6, i.e. IL-6 complexed to the IL-6R, is the only means to establish pro-inflammatory IL-6 signaling in these cells. Hper-IL-6 but not other gp130 ligands such as OSM or LIF in combination with anti-CD3/28-coated microbeads sufficed to induce rapid GzmB expression in naive CD8⁺ T cells demonstrating that IL-6 trans-signaling directly acts on T cells. The complementation of conventional vaccine protocols with Hyper-IL-6 may therefore provide an additional benefit by rapid induction of effector functions in combination with the conventional developmental process for acquisition of cytotoxic T cell effector function by matured DCs.

After several days LSEC-stimulated T cells resemble central memory T cells with respect to their localization in lymphoid tissue, their requirements for activation and generation of new effector T cells during a recall response (Bottcher et al., 2013). The rapid GzmB induction during the first 24 hours of contact with LSEC may therefore represent an intermediate developmental stage or may serve a particular function during immune responses initiated locally in the liver.

## Claims

1. A polypeptide complex comprising IL-6 and a soluble IL-6 receptor or a nucleic acid sequence encoding the same for use as an adjuvant.

2. The polypeptide complex or a nucleic acid sequence encoding the same according to claim 1 for use as an adjuvant for therapeutic or prophylactic vaccination.

3. The polypeptide complex or a nucleic acid sequence encoding the same according to any one of the preceding claims for use as mucosal adjuvant, in particular, for intranasal, intraNALT, oral, intra-rectal, intrapulmonary, intrabronchial, intrarectal, conjunctival, intra-vaginal or intra-urethral administration, administration into the milk duct of the breast or by inhalation.

4. The polypeptide complex or a nucleic acid sequence encoding the same according to claim 1 or 2 for use as systemic adjuvant, in particular, in subcutaneous, intravenous, intradermal or intramuscular administration.

5. The polypeptide complex or a nucleic acid sequence encoding the same according to any one of the preceding claims for use in a prophylactic or therapeutic vaccination against infection with an intracellular pathogen or in an anti-cancer vaccine.

6. The polypeptide complex or a nucleic acid sequence encoding the same according to claim 5 wherein said intracellular pathogen is a viral pathogen, intracellular bacterial pathogen or an intracellular parasite.

7. The polypeptide complex or a nucleic acid sequence encoding the same according to any one of the preceding claims for use in a prophylactic or therapeutic vaccine against a chronic or acute infection, in particular, chronic or acute viral infection.

8. The polypeptide complex or a nucleic acid sequence encoding the same according to any one of the preceding claims wherein the IL-6 and/or the soluble IL-6 receptor are human IL-6 and/or human soluble IL-6 receptor, in particular, human IL-6 comprising SEQ ID No. 3 or a nucleic acid sequence encoding the same and/or human soluble IL-6 receptor comprising SEQ ID No. 4 or a nucleic acid sequence encoding the same.

9. The polypeptide complex or a nucleic acid sequence encoding the same according to any one of the preceding claims whereby a flexible linker is present between the IL-6 moiety and the soluble IL-6 receptor moiety.

10. The polypeptide complex or a nucleic acid sequence encoding the same according to any one of the preceding claims for use as an adjuvant wherein the polypeptide complex is Hyper-IL-6 of SEQ ID No. 2 or a nucleic acid sequence encoding the same of Seq. ID No. 1.

11. The polypeptide complex or a nucleic acid sequence encoding the same according to any one of the preceding claims for use as a direct acting T-cell adjuvant.

12. Pharmaceutical composition comprising a compound as defined in any one of claims 1 to 11 as an adjuvant, a pharmaceutically active ingredient and, optionally, a pharmaceutically acceptable carrier, diluent, preservative, a further adjuvant other than the compounds as defined in any one of claims 1 to 11, immunemodulator and/or excipient, in particular, whereby the pharmaceutical composition is a therapeutic or prophylactic vaccine.

13. The pharmaceutical composition according to claim 12 for use as a vaccine wherein the active ingredient is an antigenic compound or a nucleic acid sequence encoding the same, in particular, an antigenic compound derived from an intracellular pathogen or cancer cell.

14. The pharmaceutical composition according to claim 12 or 13 in form of an artificial particle, in particular, in form of nanobeads or microparticles, said artificial particle comprise the polypeptide complex as defined in any one of claims 1 and 11, molecules having agonistic activity on CD28, and peptide-loaded MHC-molecules.

15. The pharmaceutical composition according to claim 14 wherein the polypeptide complex is Hyper-IL-6, the compound having agonistic activity on CD28 is an agonistic CD28 antibody, and the peptide-loaded MHC is a peptide-loaded MHC wherein the peptide is an antigenic determinant derived from an intracellular pathogen or cancer cells.

16. The polypeptide complex or a nucleic acid sequence encoding the same for use according to any one of claims 1 to 11 for activation of effector and memory T-cells in a subject, in particular, in humans.
